# EUROPEAN PATENT APPLICATION

(11) **EP 1 310 797 A2**
(43) Date of publication of application: **14.05.2003**
(21) Application number: 02256806.7
(22) Date of filing: 30.09.2002
(51) Int. Cl.: G01N 33/68

(54) **Method of monitoring neuroprotective treatment**

(30) Priority: 12.10.2001 US 328890 P
(71) Applicant: Pfizer Products Inc., Groton, Connecticut 06340 (US)
(72) Inventor: Chenard, Bertrand Leo, Pfizer Global Res. & Dev., Groton, Connecticut 06340 (US); Friedman, David Lee, Pfizer Global Res. & Dev., Groton, Connecticut 06340 (US); Kimmel, Lida Helen, Pfizer Global Res. & Dev., Groton, Connecticut 06340 (US); Nelms, Linda Fay, Pfizer Global Res. & Dev., Groton, Connecticut 06340 (US); Silber, Bernie Michael, Pfizer Global Res. & Dev., Groton, Connecticut 06340 (US); Soares, Holly Daria, Pfizer Global Res. & Dev., Groton, Connecticut 06340 (US); White, Walter Frost, Jr., Groton, Connecticut 06340 (US)
(74) Representative: England, Peter Michael

(57) **Abstract**

Methods for monitoring and evaluating the efficacy of neuroprotective treatment of a patient suffering from neurological damage by measuring the amount of at least one biomarker in a biological sample taken from the patient during or after treatment.

## Description

### FIELD OF THE INVENTION

The present invention relates to the central nervous system, particularly to methods of treating or preventing neurological damage. More particularly, the present invention relates to methods for monitoring and evaluating the efficacy of neuroprotective treatment of a patient suffering from, or at risk of suffering from, neurological damage.

### BACKGROUND OF THE INVENTION

The actual degree of neurological damage in a patient presenting with head trauma, stroke or other neurological damage is often initially unknown. The extent of neurological damage can range from minor to major, and the prognosis of any individual patient can likewise range from excellent to poor. Traditional methods of determining neurological damage, such as the Glasgow Coma Scale (GCS), are subjective measures and are considered limited in their ability to assess the true extent of neurological damage.

Interest has turned to biological markers to obtain more accurate estimates of neurological damage. One particular biological marker of interest is S-100b. "S-100" refers to a mixture of dimeric, calcium binding proteins consisting of two subunits of Mr 10,500, termed α and β. Missler et al., 1997, Stroke 28(10):1956-1960. Three isoforms are known. S-100a (αβ) is found in glial cells and melanocytes. S-100b (ββ) is present in high concentrations in glial cells and Schwann cells of the central and peripheral nervous system, as well as in Langerhans cells and cells of the anterior pituitary. S-100a0 (αα), which represents 5% of the S-100 protein in the brain, is found outside the nervous system in slow twitch muscle, heart and kidney. The biological function of S-100 proteins is not yet understood.

The appearance of S-100 proteins in peripheral blood appears to indicate both neuronal damage and increased permeability of the blood-brain barrier, and is generally considered to be a marker for brain damage (Aurell et al., 1991, Stroke 22:1254-1258; Kim et al., 1996, Stroke 27(9) 1553-1557; Westaby et al., 1996, Ann. Thorac. Surg. 61:88-92; Fassbender et al., 1997, J. Neurol. Sci. 148:101-105; Ingebrigtsen et al., 1997, J. Clin. Neurosci. 4(1):29-33; Buttner et al., 1997, Stroke 28(10):1961-1965; Abraha et al., 1997, Ann. Clin. Biochem. 34:366-370; Rosen et al., 1998, Stroke 29(2)473-477; Herrmann et al., 1999, Restor. Neurol. Neurosci. 14:109-114; Raabe et al., 1999, Neurosurgery 45(3):477-483; Wunderlich et al., 1999, Stroke 30(6):1190-1195; Hermann et al., 2000, Stroke 31:2670-2677; Herrmann et al., 2000, J. Neurotrauma 17:113-122; and U.S. Patent No. 4,654,313 to Hartman, issued March 31, 1987).

Elevated levels of S-100b in cerebrospinal fluid (CSF) or serum are seen in patients suffering from stroke, subarachnoid hemorrhage, any of various neurological diseases, subsequent to minor or severe head trauma, and in cardiac surgery patients presenting with neurological complications after circulatory arrest or cardiopulmonary bypass.

Another potentially useful marker of brain injury is neuron specific enolase (NSE), which is a dimeric, glycolytic enzyme originating from the cytoplasm of neurons and neuroendocrine cells. Another potentially useful marker of brain injury is *tau* protein, which is an intracellular protein that interacts with microtubules in neuronal axons.

U.S. Patent No. 6,235,489 B1 to Jackowski, issued May 22, 2001, describes a method for distinguishing the type of stroke suffered by a patient, comprising analyzing a blood sample from the patient to determine the level of at least four selected markers of stroke, namely, myelin basic protein (MBP), S-100 protein, NSE and a brain endothelial membrane protein such as thrombomodulin.

For the sake of optimizing neuroprotective treatment in a patient presenting with head trauma or some other type of neurological injury, or at risk of suffering from neurological damage for any reason, it is important to be able to monitor a patient's response to neuroprotective treatment.

### SUMMARY OF THE INVENTION

The present invention provides a method to monitor the response of a patient being treated for neurological damage by administration of a neuroprotective agent, comprising: (a) determining the amount of at least one biomarker in a first biological sample taken from the patient prior to an initial treatment with the neuroprotective agent; (b) determining the amount of the biomarker in at least a second biological sample taken from the patient subsequent to the initial treatment with the neuroprotective agent; and (c) comparing the amount of the biomarker in the second biological sample with the amount of the biomarker in the first biological sample; such that a detectable reduction in the amount of the biomarker in the second biological sample compared to the amount of biomarker in the first biological sample indicates that the patient is responding positively to the treatment with the neuroprotective agent. Alternatively, a positive response to treatment with the neuroprotective agent can be indicated based on an increase in the rate of reduction, or a decrease in the rate of increase, of biomarker level in the second or a subsequent biological sample in response to treatment with the neuroprotective agent.

The present invention further provides an improvement to a method for treating a patient suffering from neurological damage by administration of a neuroprotective agent, wherein the improvement comprises monitoring the level of at least one biomarker in a biological sample taken from the patient at one or more time points during treatment with the neuroprotective agent to determine whether an effective amount of the neuroprotective agent is being administered to the patient. Depending on the results of this monitoring procedure, the dose of the neuroprotective agent can be adjusted accordingly.

The present invention further provides an improvement to a method for treating a patient suffering from neurological damage by administration of a neuroprotective agent, wherein the improvement comprises monitoring the level of at least one biomarker in a biological sample taken from the patient at one or more time points during treatment with the neuroprotective agent to determine when a neuroprotective-sufficient time course of treatment with the neuroprotective agent has been completed. Completion of treatment may be indicated by the reduction of the biomarker level to a level occurring before the neurological damage occurred or otherwise to below a predetermined maximum threshold level, or by a leveling off in the rate of reduction in the amount of the biomarker in the biological sample.

The present invention further provides an improvement to a method for treating a patient suffering from neurological damage by administration of a neuroprotective agent, wherein the improvement comprises monitoring the level of at least one biomarker in a biological sample taken from the patient at one or more time points after treatment with the neuroprotective agent has been terminated so as to determine whether treatment with the neuroprotective agent needs to be restarted. The need to restart treatment may be indicated by an increase in biomarker level above a predetermined minimum threshold level, such as, e.g., if the biomarker level "spikes up" soon after a period of treatment with a neuroprotective agent has been completed.

The present invention further provides a method for identifying whether a patient will benefit from treatment with a neuroprotective agent comprising determining whether the amount of at least one biomarker in a biological sample taken from the patient prior to an initial treatment with the neuroprotective agent is above a certain predetermined minimum threshold value, such that if the amount of the biomarker in the biological sample taken from the patient is above the minimum threshold value, then the patient is primarily identified as a patient who has suffered neurological damage. This method may further comprise determining whether the amount of the biomarker in the biological sample taken from the patient prior to initial treatment with the neuroprotective agent is above a certain predetermined maximum threshold value, such that if the amount of the biomarker in the biological sample taken from the patient is above the maximum threshold value, then the patient suffering from neurological damage is secondarily identified as a patient unlikely to benefit substantially from treatment with the neuroprotective agent.

### BRIEF DESCRIPTION OF THE DRAWING

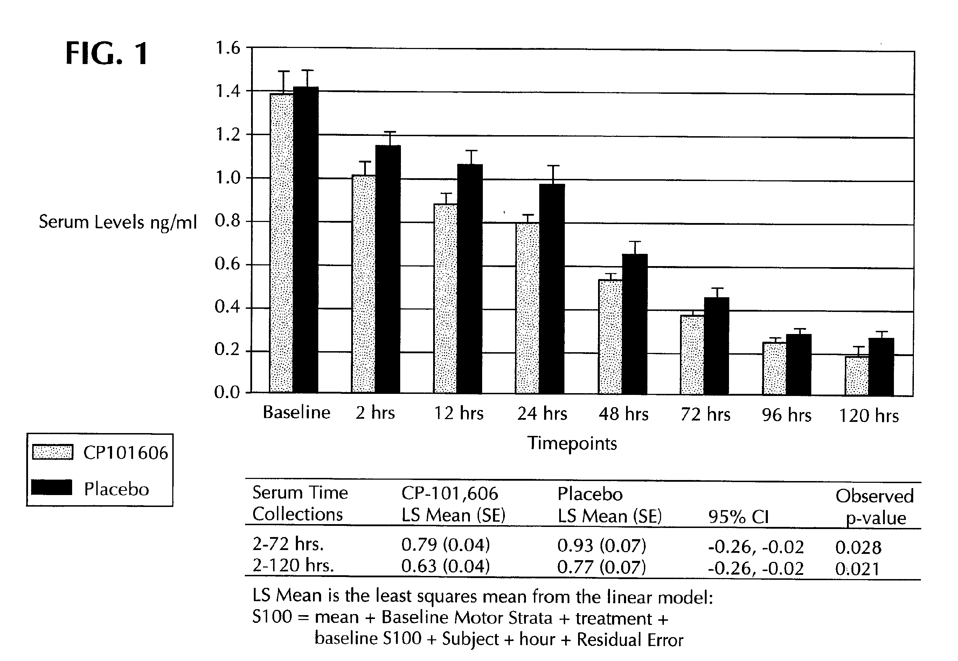

**Figure 1** presents data showing the monitoring of S-100b levels in serum samples taken from two groups of patients suffering from contusive head trauma, the first group receiving treatment with neuroprotective agent CP-101,606, and the second group receiving only a placebo.

### DETAILED DESCRIPTION OF THE INVENTION

The present invention provides methods to monitor the response of patients to neuroprotective treatment. These methods are useful: (i) to follow the response of a patient over a course of treatment with a neuroprotective agent; (ii) to determine whether the specific neuroprotective agent selected for treatment is appropriate to the patient and to the damage being treated; (iii) to determine whether the dose of the neuroprotective agent being administered is appropriate to the patient and to the damage being treated; (iv) to determine whether the type and/or amount of neuroprotective agent being administered needs to be changed over the course of the treatment period; (v) to determine when treatment is complete; and (vi) to determine whether treatment that has been terminated needs to be restarted. These methods are also useful to identify whether a patient will benefit from treatment with a neuroprotective agent.

The methods of the present invention are useful in circumstances where neurological damage to a mammalian subject, and preferably to a human patient, has occurred, is in the process of occurring, or is anticipated to occur (e.g., prior to a surgical procedure), wherein the neurological damage can be monitored by a change in the amount of a particular measurable biomarker in a biological sample that can be obtained from the subject. Where there is an anticipation of potential neurological damage, e.g., that may result from an impending surgical procedure, the monitoring can be conducted to determine when the biomarker level has been reduced to a pre-determined acceptable level, or to a "normal" pre-surgical level.

As known in the art, some patients have a reduced ability to metabolize some types of neuroprotective agents, e.g., as a result of variations in metabolism by cytochrome P-450 (e.g., CYP2D6). The methods of the present invention may be particularly useful to monitor and adjust the treatment dosages of these patients accordingly.

The methods of the present invention are also useful to monitor neurological damage, or to monitor the treatment of neurological damage, in other animals such as primates other than humans, or in companion animals such as dogs or cats, or in horses.

As used herein, "neurological damage" includes any neurological damage caused by any condition, disease or event resulting in a partial or complete impairment in the supply of blood, oxygen or glucose to the CNS, or by a traumatic injury to the CNS. Non-limiting examples of such a condition, disease or event include cerebral ischemia, cerebral infarction, cerebral vasospasm, traumatic head injury, traumatic spinal cord injury, hemorrhage (such as subarachnoid hemorrhage, cerebral hemorrhage or aneurysmal hemorrhage), asphyxia (e.g., perinatal asphyxia), cardiac arrest, cardiac infarction, hypoxia or anoxia (e.g., from drowning, suffocation, anesthesia administered during surgical procedures, pulmonary surgery, cardiac bypass, or use of a heart-lung machine), hypoglycemia, reperfusion injury, a progressive pathological condition (e.g., Alzheimer's Disease, Parkinson's Disease, Huntington's Disease, Multiple Sclerosis, HIV-related neurodegeneration or cerebellar degeneration), seizure, glioblastoma, polyneuropathy, hydrocephalus, encephalitis, meningitis, epilepsy and schizophrenia, among others known in the art.

The methods of the present invention are particularly useful where the neurological damage is caused by cerebral ischemia or traumatic head or spinal cord injury. The methods of the present invention will also be particularly useful where the neurological damage is caused by hypoxia or anoxia. Hypoxia as used herein is defined as a condition where the level of oxygen in a tissue is reduced below normal levels. Hypoxia can result from a variety of circumstances, including any event that disrupts normal blood flow, or that impairs normal oxygenation of the blood, or that impairs the normal ability of blood to carry oxygen to the tissues. In a non-limiting embodiment, hypoxia can be caused by ischemia, hemorrhage (e.g., subarachnoid hemorrhage, cerebral hemorrhage or aneurysmal hemorrhage), cardiac infarction or cardiac arrest, drowning, anesthesia administered during a surgical procedure, the use of a heart-lung machine, or impaired lung function. Impaired lung function can be caused by emphysema, cigarette smoking, chronic bronchitis, asthma, infectious agents, pneumonitis, and the like. The term "anoxia" is often used interchangeably with the term "hypoxia", but generally refers to a greater decrease, and even a complete depletion, of oxygen in a tissue.

The methods of the present invention are also particularly useful where the neurological damage has occurred, or may occur, as the result of a surgical procedure such as, e.g., open-heart surgery including but not limited to coronary artery bypass graft (CABG) or any other cardiac procedure such as, e.g., angiography or angioplasty, or pulmonary surgery, etc. For example, both short-term and long-term cognitive deficits have been documented in patients after CABG (McKhann *et al*., 1998, *Ann. Thorac. Surg.* 63:510-5). In one study, only 12% of patients showed no decline across eight cognitive domains (verbal memory, visual memory, language, attention, vasoconstruction, psychomotor speed, motor speed, and executive function).

Neurological damage may be identified by a variety of diagnostic tests known in the art, which detect impairment of neurological function. Examples of such neurological tests include the Glascow Outcome Scale (GOS), the Glascow Coma Scale (GCS), the Disability Rating Scale (DRS) and the NIH Stroke Scale (NIHSS), which can be carried out using known methods. Other methods for detecting neurological damage include CAT scans and determining intracranial pressure.

Neuroprotective agents can be administered to patients: (i) to treat neurological damage that has already occurred; (ii) to prevent further neurological damage caused during the subsequent cascade of biochemical and cellular responses that often follow a partial or complete impairment in the supply of blood, oxygen or glucose, or a traumatic injury, to the CNS; or (iii) as a prophylactic treatment to prevent future neurological damage, e.g., that might result from an upcoming surgical procedure.

The present invention is based on the identification of specific biomarkers of neurological damage, and the observation that efficacy of neuroprotective treatment can be monitored by tracking changes in the levels of one or more such biomarkers in response to such treatment. A biomarker useful according to the methods of the present inventions can be a molecule that is present in undamaged tissues or cells and which, upon neurological injury or insult, is released from or secreted by the tissues or cells of the CNS into a biological tissue or fluid from which a biological sample can be obtained from a patient, such as, e.g., cerebrospinal fluid (CSF) or across the blood-brain barrier into non-CNS tissues such as the circulatory or lymph system, or into the saliva, or as excreted in the urine or in feces. Alternatively, the biomarker can be a molecule produced *de novo* in response to neurological injury or insult, and which accumulates in a biological tissue or fluid from which a biological sample can be obtained from a patient, such as, e.g., the CSF or non-CNS tissues such as the circulatory or lymph systems, or as excreted in the urine.

A preferred biomarker is any biological molecule that can be detected and quantified in a biological sample using standard biochemical assay methods, where the presence and/or quantity of the biomarker in the biological sample: (i) can be correlated with either the degree or ongoing progression of neurological damage; (ii) can be used to predict a patient's prognosis; (iii) can be used to select an appropriate neuroprotective treatment; or (iv) can be used to monitor the efficacy and progress of neuroprotective treatment.

In one embodiment, the biomarker is a member of the S-100 family of proteins. In a more preferred embodiment , the biomarker is S-100b (ββ) or S-100a (αβ), and most preferably S-100b (ββ) (see, e.g., Missler et al., 1997, *supra*; Aurell et al., 1991, *supra*; Kim et al., 1996, *supra*; Westaby et al., 1996, *supra*; Fassbender et al., 1997, *supra*; Ingebrigtsen et al., 1997, *supra*; Buttner et al., 1997, *supra*; Abraha et al., 1997, *supra*; Rosen et al., 1998, *supra;* Herrmann et al., 1999, *supra;* Raabe et al., 1999, *supra;* Wunderlich et al., 1999, *supra;* Hermann et al., 2000, Stroke 31:2670-2677; Herrmann et al., 2000, J. Neurotrauma 17:113-122; U.S. Patent No. 4,654,313 to Hartman, issued March 31, 1987; and WO 00/52476).

In another embodiment, the biomarker is neuron-specific enolase (NSE) (see, e.g., Missler et al., 1997, *supra*; Fassbender et al., 1997, *supra;* Herrmann et al., 1999, *supra;* Wunderlich et al., 1999,*supra*; Herrmann et al., 2000, J. Neurotrauma 17:113-122; and WO 00/52476).

In another embodiment, the biomarker is glial fibrillary acidic protein (GFAP) (see, e.g., Aurell et al., 1991, supra; Hermann et al., 2000, Stroke 31:2670-2677).

In another embodiment, the biomarker is *tau* protein (see, e.g., WO 99/45393 by the University of Cincinnati, published September 10, 1999).

In another embodiment, the biomarker is haptoglobin (see, e.g., U.S. Patent No. 5,429,947 to Merril et al., issued July 4, 1995; U.S. Patent No. 4,103,687 to Ishii, issued August 1, 1978).

In another embodiment, the biomarker is glutamate. Glutamate is a highly abundant excitatory neurotransmitter in the brain. Following severe head injury, local intercellular glutamate concentrations are believed to increase rapidly leading to excitatory cell death. The concentration of glutamate that appears in the CSF following severe head injury may reflect the extent of neuronal damage incurred.

In another embodiment, the biomarker is creatine kinase (see, e.g., U.S. Patent No. 5,817,467 to Aoyama et al., issued October 6, 1998).

In another embodiment, the biomarker is F2-isoprostane (see, e.g., U.S. Patent No. 5,891,622 to Morrow et al., issued April 6, 1999).

In another embodiment, the biomarker is myelin basic protein (MBP) or thrombomodulin (see, e.g., WO 00/52476).

In carrying out any of the methods of the present invention, the levels of either a single biomarker, or a panel of two or more different biomarkers, e.g., both S-100b and NSE, can be assayed. Assay of more than one biomarker may serve to increase the accuracy of determining the degree of neurological damage or of monitoring the response of the patient to neuroprotective treatment. Measurement of a plurality of biomarkers can be carried out by assaying the different biomarkers in either the same biological sample or in different biological samples taken from the same patient.

The present invention thus provides a method to monitor the response of a patient being treated for neurological damage by administration of a neuroprotective agent, comprising: (a) determining the amount of at least one biomarker in a first biological sample taken from the patient prior to an initial treatment with the neuroprotective agent; (b) determining the amount of the biomarker in at least a second biological sample from the patient subsequent to the initial treatment with the neuroprotective agent; and (c) comparing the amount of the biomarker present in the second biological sample with the amount of the biomarker present in the first biological sample; such that a detectable reduction, or prevention or slowing of an increase, in the amount of the biomarker present in the second biological sample, and/or in any subsequent biological samples, compared to the amount of biomarker present in the first biological sample indicates that the patient is responding positively to the treatment with the neuroprotective agent. Alternatively, where the amount of biomarker in a second or subsequent biological sample would tend to decrease naturally without treatment with a neuroprotective agent, e.g., as the response to the initial neurological damage subsides, a positive response to treatment with the neuroprotective agent can be indicated by a detectable increase in the rate of reduction of the amount of the biomarker present in the second or subsequent biological sample compared to the rate of reduction in the amount of biomarker that would be expected to occur without treatment with a neuroprotective agent (i.e., in a control biological sample) over a comparable time course.

In contrast, a lack of reduction (e.g., either no detectable change or an increase in the total amount) in the amount of the biomarker in the second or subsequent biological sample compared to the amount of the biomarker in the first biological sample may indicate that the patient is not responding positively to the treatment with the neuroprotective agent. Alternatively, in appropriate circumstances, a lack of increase in the rate of reduction in the amount of the biomarker in the second or subsequent biological sample compared to the rate of reduction in the amount of biomarker that would be expected to occur in a control biological sample over a comparable time course may indicate that the patient is not responding positively to the treatment with the neuroprotective agent.

In a preferred embodiment, the biomarker is a member of the S-100 family of proteins such as S-100b. In another preferred embodiment, the biomarker is NSE. In another preferred embodiment, the biomarker is *tau* protein. In another preferred embodiment, the biomarker is haptoglobin.

This method requires that at least two biological samples are taken from the patient at different time points. The first sample is typically obtained prior to an initial treatment with the neuroprotective agent, e.g., upon initial presentation at a physician's office or in a hospital emergency room setting as a result, e.g., of a traumatic head or spinal cord injury, or in response to the suspected occurrence of a neurological event such as a stroke. A second sample is preferably obtained, and any subsequent samples are preferably obtained, after neuroprotective treatment has begun. In this method, the biomarker is monitored to determine: (i) if the amount of the biomarker is decreasing, (ii) if the rate of decrease in the amount of the biomarker is increasing, or (iii) if the amount of biomarker is stabilizing, any one of which may indicate that the patient is responding positively to the neuroprotective treatment depending upon the specific circumstances. If the biomarker level remains elevated over normal levels, or if the rate of decrease of the biomarker level is not sufficiently high, the neuroprotective treatment can be modified to a more aggressive protocol, such as by increasing the dosage or the number of treatments, or by changing the neuroprotective agent being administered to a more effective agent, or by combining the neuroprotective agent being used in the treatment with one or more other neuroprotective agents or therapies, or some combination thereof.

The present invention further provides an improvement to a method for treating a patient suffering from neurological damage by administration of a neuroprotective agent, wherein the improvement comprises monitoring the level of at least one biomarker in a biological sample taken from the patient at one or more time points during treatment with the neuroprotective agent so as to determine whether an effective amount of the neuroprotective agent is being administered to the patient. An "effective amount of the neuroprotective agent" is being administered to the patient if the level of the biomarker in the biological sample detectably decreases, or if a previously observed rate of increase in the level of biomarker detectably slows, levels off, or is reversed, or if a previously observed rate of decrease in the level of the biomarker increases, in response to administration of the neuroprotective agent.

The present invention further provides an improvement to a method for treating a patient suffering from neurological damage by administration of a neuroprotective agent, wherein the improvement comprises monitoring the level of at least one biomarker in a biological sample taken from the patient at one or more time points during treatment with the neuroprotective agent so as to determine when a neuroprotective-sufficient time course of treatment with the neuroprotective agent has been completed. In a preferred embodiment, a "neuroprotective-sufficient time course of treatment with the neuroprotective agent has been completed" when the level of biomarker detectably decreases below a maximum threshold level that has been set as an indicator of neurological damage. For example, an "effective amount of the neuroprotective agent" has been administered if the level of the biomarker in the biological sample remains below a maximum threshold level for a substantial period of time such as, e.g., where serum levels of S-100b remain below 0.2 ug/L, or where serum levels of NSE remain below 10 ng/L for a substantial period of time. Examples of substantial periods of time include more than 24 hours, or more than 48 hours, or more than 72 hours, or more than 120 hours.

In a preferred embodiment, a "maximum threshold level" is the uppermost level of any particular biomarker that is normally found in a patient that is not experiencing an increase in the biomarker level as the result of current or recent neurological damage. For example, a normal subject that is healthy and is not currently experiencing any other neurodegenerative disease or condition would typically have a maximum threshold serum level of S-100b of about 0.2 µg/L, and values above this level would tend to indicate neurological damage. Where, for example, a subject is also suffering from another neurodegenerative disease or condition in addition to the specific traumatic event being treated, the "normal" maximum threshold serum level of S-100b could be higher than 0.2 ug/L, and this can be determined and should be taken into account by the attending medical practitioner.

The present invention further provides an improvement to a method for treating a patient suffering from neurological damage by administration of a neuroprotective agent, wherein the improvement comprises monitoring the level of at least one biomarker in a biological sample taken from the patient at one or more time points after treatment with the neuroprotective agent has been terminated so as to determine whether treatment with the neuroprotective agent needs to be restarted. The need to restart treatment with a neuroprotective agent is typically indicated when the level of the biomarker in a biological sample taken from the patient "spikes up", or otherwise detectably begins to rise above a threshold level after treatment with the neuroprotective agent has been reduced or terminated. Such a threshold level can be a maximum threshold level that has been set as an indicator of neurological damage, or can be a level unique to the particular patient as determined based on previous biomarker levels measured in biological samples taken from that patient, such as, e.g., prior to or during initial treatment with the neuroprotective agent, or prior to a surgical procedure.

The type of biological sample from which the amount of biomarker is determined will depend on a variety of factors such as the particular biomarker, where and when it is synthesized, where the biomarker may be stored in the tissues, and into what biological tissue or fluid it may be released or otherwise accumulate. Generally, the biological sample will be selected from the group consisting of blood, a blood component such as serum or plasma, cerebrospinal fluid (CSF), saliva and urine. In a preferred embodiment, the biological sample will be blood, serum, plasma or CSF, and most preferably blood, serum or plasma. Where more than one biomarker is analyzed, the analysis can be conducted on the same or different biological samples obtained from the patient.

The amount of the biomarker(s) in a biological sample can be determined using those standard techniques currently known in the art or to be developed in the future. For example, each biomarker can be assayed using biomarker-specific antibodies and immunological methods known in the art. Any appropriate immunoassay method can be used, including radioimmunoassays, sandwich enzyme-linked immunoassays, competitive binding assays, homogeneous assays, and heterogeneous assays. Alternatively, the amount of biomarker(s) can be determined using other techniques such as magnetic resonance spectroscopy, HPLC or mass spectrometry. In any case, the assay method selected should be sensitive enough to be able to measure the particular biomarker in a concentration range from normal values found in healthy patients to elevated levels indicating neurological damage. The assay can be carried out in various formats including, e.g., in a microtiter plate format, using automated immunoassay analyzers known in the art.

S-100b levels in a biological sample can be determined using any of the techniques described in Missler et al., 1997, *supra;* Aurell et al., 1991, *supra;* Kim et al., 1996, *supra*; Westaby et al., 1996, *supra;* Fassbender et al., 1997, *supra;* Ingebrigtsen et al., 1997, *supra;* Buttner et al., 1997, *supra;* Abraha et al., 1997, *supra;* Rosen et al., 1998, *supra;* Herrmann et al., 1999, *supra;* Raabe et al., 1999, *supra;* Wunderlich et al., 1999, *supra;* Hermann et al., 2000, Stroke 31:2670-2677; Herrmann et al., 2000, J. Neurotrauma 17:113-122; U.S. Patent No. 4,654,313 to Hartman, issued March 31, 1987; or WO 00/52476. Healthy men and women typically show S-100b levels in serum below about 0.15 to about 0.20 µg/L and levels in CSF of less than about 5.0 µg/L (95 percentile). As used herein the term "about" refers to the specifically stated numerical value plus or minus 10%. Blood or serum levels of S-100b of more than about 0.20 µg/L, and CSF levels of more than about 5.0 µg/L, will usually indicate the occurrence of neurological damage, with higher levels of S-100b correlating with increasing levels of neurological damage, although allowance should be made for variations in these values between different patients.

Levels of S-100b can be measured in a biological sample of a patient's blood or CSF using a commercially available immunoradiometric assay or luminometric immunoassay kit such as those available from Byk-Sangtec Diagnostica GmbH & Co. (Dietzenbach, Germany), which are based on a two-site immunoluminometric sandwich assay using the commercially available LIAISON® Sangtec® S-100 assay system. In this assay system, paramagnetic particles are coated with two anti-S100b monoclonal antibodies directed to different epitopes, and a secondary anti-S100b monoclonal antibody labeled with an isoluminol derivative is provided as detection antibody. The paramagnetic particles, assay buffer, and biological sample are first incubated and unbound material is removed by a wash cycle. Detection antibody is added and, after a second incubation, unbound detection antibody is removed by a second wash cycle. Subsequently, starter reagents that activate the chemoluminscent reaction are added and the S-100b concentration is determined via the chemiluminescence reaction induced by the previously added reagents. The light signal is measured in relative light units (RLUs) and is directly proportional to the amount of S-100b protein in the sample. The detection limit of the LIAISON® S-100 assay system is 0.02 µg/L (mean value ± 3 std deviations), and the measuring range is from about 0.02 µg/L to about 30 µg/L. The LIAISON® S-100 assay system can be calibrated using lyophilized reagents according to instructions provided by the manufacturer.

Levels of NSE in a biological sample can be determined using any of the techniques described in Missler et al., 1997, *supra;* Fassbender et al., 1997, *supra*; Herrmann et al., 1999, *supra*; Wunderlich et al., 1999,*supra*; or Herrmann et al., 2000, J. Neurotrauma 17:113-122; or WO 00/52476. Healthy men and women typically show NSE levels in serum below about 10-12.5 µg/L and in CSF below about 20 ug/L (95 percentile). As used herein the term "about" refers to the specifically stated numerical value plus or minus 10%. Blood or serum levels of NSE of more than about 12.5 µg/L, and CSF levels of more than about 20 ug/L, will usually indicate the occurrence of neurological damage, with higher levels of NSE correlating with increasing levels of neurological damage, although allowance should be made for variations in these values between different patients.

Levels of NSE can be measured in a biological sample of a patient's blood or CSF using a commercially available immunoradiometric assay or luminometric immunoassay kit from Byk-Sangtec Diagnostica GmbH & Co. (Dietzenbach, Germany), such as, e.g., LIAISON® NSE, which is a two-site immunoluminometric sandwich assay in which tracer antibody and immobilized antibody react simultaneously with NSE present in patient samples and standards. After incubation, excess tracer is removed by a wash cycle. Subsequently, the starter reagents are added. The NSE concentration is determined via a chemiluminescence reaction induced by trigger reagents. The light signal measured in relative light units (RLUs) is directly proportional to the amount of NSE in the sample. The detection limit of the LIAISON® NSE assay system is 0.04 µg/L (mean value ± 2 std deviations), and the measuring range is from about 0.04 µg/L to about 200 µg/L. The LIAISON® NSE S-100 assay system can be calibrated using lyophilized reagents and according to instructions provided by the manufacturer.

Levels of *tau* protein in a biological sample can be determined using any of the techniques described in WO 99/45393. Healthy men and women below the age of 40 typically show *tau* protein levels in CSF below about 200 pg/mL. As used herein the term "about" refers to the specifically stated numerical value plus or minus 10%. CSF levels of *tau* protein of more than about 200 pg/mL will usually indicate the occurrence of neurological damage, with higher levels of *tau* protein correlating with increasing levels of neurological damage, although allowance should be made for variations in these values between different patients, and patients older than 40.

Immunoassay kits and reagents specific to measure tau protein in biological samples are commercially available, e.g., from Innogenetics N.V., Zwijnaarde, Belgium. For example, tau protein levels in CSF can be determined using the Innotest™ hTAU antigen kit using an enzyme immunoassay format, wherein test samples are incubated with a pair of biotinylated *tau*-specific monoclonal antibodies recognizing different epitopes. Streptavidin-conjugated horseradish peroxidase binds to the biotin and in the presence of substrate and chromogen produces the colored product, wherein the intensity of the color is proportional to the tau protein concentration in the sample. The detection limit of the Innotest™ hTAU antigen kit is 59.3 pg/ml (mean value ± 4 std deviations), and the measuring range is from about 50 pg/ml to about 1200 pg/ml. The Innotest™ hTAU antigen kit test results can be calibrated using lyophilized reagents and according to instructions provided by the manufacturer.

Levels of GFAP in a biological sample can be determined using any of the techniques described in Aurell et al., 1991, supra; or Hermann et al., 2000, Stroke 31:2670-2677. Immunoassay kits and reagents specific to measure GFAP in biological samples are commercially available, e.g., from Innogenetics N.V., Zwijnaarde, Belgium. Healthy men and women typically show GFAP levels in CSF below about 0.4 ug/L. As used herein the term "about" refers to the specifically stated numerical value plus or minus 10%. CSF levels of GFAP of more than about 0.4 µg/L will usually indicate the occurrence of neurological damage, with higher levels of GFAP correlating with increasing levels of neurological damage, although allowance should be made for variations in these values between different patients.

Glutamate levels in a biological sample can be determined using standard amino acid analysis techniques or modifications thereof. For example, glutamate levels may be monitored in CSF using a modification of the Waters Corporation AccQ-Tag amino acid analysis kit (Waters Corporation, Milford Massachusetts). Primary and secondary amines in a sample are converted to stable, fluorescent derivatives by reaction with 6-aminoquinolyl-N-hydroxysuccinimidyl carbamate (AQC). The reverse-phase separation of these fluorescent amine derivatives is optimized to produce a highly resolved derivatize glutamate peak with low retention time, without regard for resolution of any other derivatives. Healthy men and women normally show glutamate levels in CSF below about 2.0 µM. As used herein the term "about" refers to the specifically stated numerical value plus or minus 10%. CSF levels of glutamate of more than about 2.0 µM will usually indicate the occurrence of neurological damage, with higher levels of glutamate correlating with increasing levels of neurological damage, although allowance should be made for variations in these values between different patients.

Creatine kinase levels in a biological sample can be determined using any of the techniques described in U.S. Patent No. 5,817,467.

Levels of isoprostane in a biological sample can be determined using any of the techniques described in U.S. Patent No. 5,891,622.

Levels of myelin basic protein (MBP), or a brain endothelial membrane protein such as thrombomodulin, in a biological sample can be determined using any of the techniques described in U.S. Patent No. 6,235,489.

As used herein, the phrase "a threshold amount of the biomarker in the biological sample" refers to that amount or concentration of the particular biomarker in a biological sample, wherein the amount of the biomarker is significantly higher statistically than would normally be expected to be present in a biological sample obtained from a control subject that is similar in all important aspects to the patient, but that is not currently suffering from specific neurological damage. The specific threshold amount depends upon the particular biomarker. For example, for S-100b, apparently healthy individuals without neurological damage typically have serum levels of less than about 0.2 µg/L and CSF levels of less than about 5.0 µg/L. For NSE, apparently healthy individuals without neurological damage typically have serum levels of less than about 10 to about 12.5 µg/L, and CSF levels of less than about 20 ug/L. For GFAP, apparently healthy individuals without neurological damage typically have CSF levels of less than about 0.4 µg/L. For *tau* protein, apparently healthy individuals without neurological damage typically have CSF levels of less than about 200 pg/mL. For example, in the CSF, *tau* protein levels in apparently healthy individuals younger than 60 years of age without neurological damage tend to be less than about 119.4 pg/mL and, for individuals 60 years of age and older without neurological damage, *tau* protein levels tend to be less than about 171.1 pg/L. For haptoglobin, apparently healthy individuals without neurological damage typically do not have detectable levels in the CSF. Detectable haptoglobin in the CSF may be indicative of the failure of the blood-brain barrier. Threshold levels indicating neurological damage would be any values significantly higher statistically than any of these normal levels.

The neuroprotective agent used according to the methods of the present invention is any chemical compound, including any neuroprotective pharmaceutically acceptable salt or solvate thereof, or any neuroprotective pharmaceutical composition thereof, that can protect the integrity and function of, or treat damage to, any of the tissues or cells of the CNS, and particularly the neurons, glial cells, or endothelial cells, from a condition, disease or event that would otherwise result in damage to such tissues or cells or to the integrity of the blood-brain barrier. Such a neuroprotective agent serves to prevent, reduce or treat the damage that would otherwise occur to such tissues or cells caused by such condition, disease or event. Alternatively, the neuroprotective agent may be a neuroprotective therapy such as hypothermic treatment administered to a patient in response to a condition, disease or event that would otherwise increase the risk of neurological damage, which therapy is intended to protect the integrity and function of the tissues and cells of the CNS from damage that would otherwise result.

Neuroprotective agents used according to a method of the present invention can provide neuroprotection by any method or mechanism currently known or to be developed in the future. For example, neuroprotection can be provided by administration of a neuroprotection-effective amount of any of the following types of agents, either alone or in combination: excitatory amino acid receptor antagonists, such as NMDA receptor antagonists, AMPA receptor antagonists, and kainic acid receptor antagonists; metabotropic glutamate receptor agonists or antagonists; GABA receptor antagonists; NAALDase enzyme inhibitors; calpain inhibitors; p38 mitogen-activated protein kinase (MAPK) inhibitors; estrogen enantiomers and derivatives; modulators of nitric oxide production; calmodulin inhibitors; adenosine receptor modulators; purine receptor antagonists; neutrophil inhibitory factors (NIF); thrombolytic agents like tissue plasminogen activator or streptokinase; prosaposin receptor activity stimulators; or synthetic oxygen carriers; among others.

Non-limiting examples of neuroprotective agents, and methods of making and using them, are provided in the following documents among others: U.S. Patent No. 4,690,931 to Wick et al., issued September 1, 1987; U.S. Patent No. 5,185,343 to Chenard, issued February 9, 1993; U.S. Patent No. 5,272,160 to Chenard, issued December 21, 1993; U.S. Patent No. 5,306,723 to Chenard, issued April 26, 1994; U.S. Patent No. 5,338,754 to Chenard, issued August 16, 1994; U.S. Patent No. 5,356,905 to Butler, issued October 18, 1994; U.S. Patent No. 5,373,018 to Cugola et al., issued December 13, 1994; U.S. Patent No. 5,391,742 to Chenard, issued February 21, 1995; U.S. Patent No. 5,455,250 to Chenard, issued October 3, 1995; U.S. Patent No. 5,455,279 by Lipton, issued October 3, 1995; U.S. Patent No. 5,510,367 to Cugola et al., issued April 23, 1996; U.S. Patent No. 5,514,680 to Weber et al., issued May 7, 1996; U.S. Patent No. 5,527,912 to Chenard, issued June 18, 1996; U.S. Patent No. 5,607,973 to Theriault, issued March 4, 1997; U.S. Patent No. 5,620,978 to Cai et al., issued April 15, 1997; U.S. Patent No. 5,620,979 to Weber et al., issued April 15, 1997; U.S. Patent No. 5,622,952 to Weber et al, issued April 22, 1997; U.S. Patent No. 5,631,373 to Cai et al., issued May 20, 1997; U.S. Patent No. 5,654,302 to Chenard, issued August 5, 1997; U.S. Patent No. 5,661,033 to Ho et al., issued August 26, 1997; U.S. Patent No. 5,661,150 to Shirasaki et al., issued August 26, 1997; U.S. Patent No. 5,710,168 to Chenard, issued January 20, 1998; U.S. Patent No. 5,716,961 to Sands, issued February 10, 1998; U.S. Patent No. 5,728,728 to Kozachuk, issued March 17, 1998; U.S. Patent No. 5,744,483 to Butler et al., issued April 28, 1998; U.S. Patent No. 5,767,130 to Olney, issued June 16, 1998; U.S. Patent No. 5,801,183 to Keana et al., issued September 1, 1998; U.S. Patent No. 5,824,662 to Slusher et al., issued October 20, 1998; U.S. Patent No. 5,827,832 to Sandage Jr. et al., issued October 27, 1998; U.S. Patent No. 5,834,465 to Olney, issued November 10, 1998; U.S. Patent No. 5,854,217 to Maccecchini, issued December 29, 1998; U.S. Patent No. 5,863,916 to Cai et al., issued January 26, 1999; U.S. Patent No. 5,880,138 to Heinz et al., issued March 9, 1999; U.S. Patent No. 5,889,026 to Alanine et al., issued March 30, 1999; U.S. Patent No. 5,902,815 to Olney et al., issued May 11, 1999; U.S. Patent No. 5,906,996 to Murphy, issued May 25, 1999; U.S. Patent No. 5,925,634 to Olney, issued July 20, 1999; U.S. Patent No. 5,935,606 to Sagen, issued August 10, 1999; U.S. Patent No. 5,939,407 to Landfield, issued August 17, 1999; U.S. Patent No. 5,939,432 to Baraldi, issued August 17, 1999; U.S. Patent No. 5,952,344 to Alanine et al., issued September 14, 1999; U.S. Patent No. 5,952,389 to Fogel, issued September 14, 1999; U.S. Patent No. 5,958,919 to Olney et al., issued September 28, 1999; U.S. Patent No. 5,977,107 to Cai et al., issued November 2, 1999; U.S. Patent No. 6,004,946 to Slusher et al., issued December 21, 1999; U.S. Patent No. 6,008,233 to Andino et al, issued December 28, 1999; U.S. Patent No. 6,013,672 to Ye et al., issued January 11, 2000; U.S. Patent No. 6,015,824 to Alanine et al., issued January 18, 2000; U.S. Patent No. 6,028,080 to Ackermann et al., issued February 22, 2000; U.S. Patent No. 6,034,134 to Gold et al., issued March 7, 2000; U.S. Patent No. 6,046,213 to Chenard et al., issued April 4, 2000; U.S. Patent No. 6,048,865 to Baraldi, issued April 11, 2000; U.S. Patent No. 6,063,819 to Marangos et al., issued May 16, 2000; U.S. Patent No. 6,083,941 to Farb, issued July 4, 2000; U.S. Patent No. 6,096,744 to Kornberg et al., issued August 1, 2000; U.S. Patent No. 6,110,894 to Maccecchini, issued August 29, 2000; U.S. Patent No. 6,124,317 to Bigge et al., issued September 26, 2000; U.S. Patent No. 6,124,323 to Bigge et al., issued September 26, 2000; U.S. Patent No. 6,130,234 to Bigge et al., issued October 10, 2000; U.S. Patent No. 6,147,075 to Cai et al., issued November 14, 2000; U.S. Patent No. 6,153,624 to Alanine et al., issued November 28, 2000; U.S. Patent No. 6,159,958 to Meyerhoff et al., issued December 12, 2000; U.S. Patent No. 6,172,041 to McCabe et al., issued January 9, 2001; U.S. Patent No. 6,180,597 to Liao, issued January 30, 2001; U.S. Patent No. 6,197,788 to Fletcher et al., issued March 6, 2001; and U.S. Patent No. 6,218,404 to Bigge et al., issued April 17, 2001.

Further non-limiting examples of neuroprotective agents, and methods of making and using them, are also provided in the following documents, among others: EP 0 202 164 A1 by Synthelabo, published November 20, 1986; EP 0 459 830 A1 by The Wellcome Foundation, Ltd., published December 4, 1994; EP 0 648 744 A1 by F. Hoffmann-La Roche AG, published April 19, 1995; EP 0 824 098 A1 by F. Hoffmann-La Roche AG, published February 18, 1998; and EP 0 846 683 A1 by F. Hoffmann-La Roche AG, published June 10, 1998; WO 90/14088 by Pfizer Inc., published November 29, 1990; WO 92/18502 by Pfizer Inc., published October 29, 1992; WO 96/06081 by Pfizer Inc., published February 29, 1996; WO 96/37226 by Pfizer Inc., published November 28, 1996; WO 97/07098 by Pfizer Inc., published February 27, 1997; WO 97/23202 by State of Oregon, published July 3, 1997; WO 97/23214 by Wamer-Lambert Co., published July 3, 1997; WO 97/23215 by Warner-Lambert Co., published July 3, 1997; WO 97/23216 by Wamer-Lambert Co., published July 3, 1997; WO 97/23458 by Warner-Lambert Co., published July 3, 1997; WO 97/32581 by F. Hoffmann-La Roche AG, published September 12, 1997; WO 97/32858 by Fujisawa Pharm. Co., published September 12, 1997; WO 97/46877 by The Univ. Of Edingburgh, published December 11, 1997; WO 98/03191 by Neurotrauma Therapeutics, Inc., published January 29, 1998; WO 98/18793 by Merck Patent GMBH, published May 7, 1998; WO 99/21541 by Alliance Pharmaceutical Corp., published May 6, 1999; WO 99/25683 by Klinikum der Albert-Ludwigs-Universitat Freiburg, published May 27, 1999; WO 99/31051 by Cerebrus Ltd., published June 24, 1999; WO 99/33849 by Guildford Pharmaceuticals Inc., published July 9, 1999; WO 99/44610 by Merck Sharp & Dohme Ltd., published September 10, 1999; WO 99/44640 by Merck Sharp & Dohme Ltd., published September 10, 1999; WO 99/51565 by Advanced Medicine, Inc., published October 14, 1999; WO 00/11204 by The Johns Hopkins University School of Medicine, published March 2, 2000; WO 00/12488 by Glaxo Group Ltd., published March 9, 2000; WO 00/14113 by Myelos Corporation, published March 16, 2000; WO 00/18758 by Mitsubishi Chem. Corp., published April 6, 2000; WO 00/24395 by Ikonomidou, published May 4, 2000; WO 00/43039 by During, published July 27, 2000; WO 00/44371 by Vemalis Research Ltd., published August 3, 2000; WO 00/50058 by Keep et al., published August 31, 2000; WO 00/51586 by NPS Pharmaceuticals, Inc., published September 8, 2000; WO 00/56403 by The Brigham and Women's Hospital, Inc., published September 28, 2000; WO 00/56711 by Sumitomo Pharmaceuticals Co. Ltd., published September 28, 2000; WO 00/57879 by Reisberg et al., published October 5, 2000; WO 00/61126 by Eli Lilly, published October 19, 2000; WO 00/62771 by The UAB Res. Foundation, published October 26, 2000; WO 00/64911 by Georgetown University, published November 2, 2000; WO 00/67751 by Merck & Co., Inc., published November 16, 2000; WO 00/67755 by Merck & Co., Inc., published November 16, 2000; WO 00/71534 by Abbott Laboratories, published November 30, 2000; WO 00/75109 by F. Hoffmann-La Roche AG, published December 14, 2000; WO 01/01986 by Lipton, published January 11, 2001; WO 01/02387 by Fujisawa Pharmaceutical Co. Ltd., published January 11, 2001; WO 01/02566 by Neurocrine Biosciences, Inc., published January 11, 2001; WO 01/05404 by C.N.R.S., published January 25, 2001; WO 01/05790 by Astrazeneca AB, published January 25, 2001; WO 01/05963 by McGill University, published January 25, 2001; WO 01/07022 by Vemalis Research Ltd., published February 1, 2001; WO 01/07043 by Vemalis Research Ltd., published February 1, 2001; WO 01/08692 by Imperial College of Sci., Tech. and Med., published February 8, 2001; and WO 01/10430 by Univ. Florida Res. Foundation, Inc., published February 15, 2001.

In a preferred embodiment, the neuroprotective agent is an NMDA receptor antagonist, and more preferably an NR2B-selective NMDA antagonist. Examples of such antagonists are described in Chenard and Menniti, 1999, Curr. Pharm. Design 5:381-404, which is incorporated herein by reference in its entirety, and which describes CP-101,606, ifenprodil and eliprodil, among others.

An NR2B-selective NMDA antagonist that can be used in the methods of the present invention includes a compound of formula I 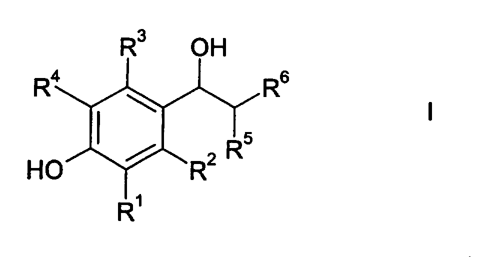 or pharmaceutically acceptable acid addition salt or solvate thereof, wherein:
(a) R² and R⁵ are taken separately and R¹, R², R³ and R⁴ are each independently hydrogen, (C₁-C₆) alkyl, halo, CF₃, OH or OR⁷ and R⁵ is methyl or ethyl; or
(b) R² and R⁵ are taken together and are 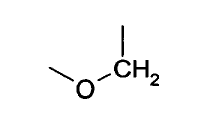 forming a chroman-4-ol ring, and R¹, R³ and R⁴ are each independently hydrogen, (C₁-C₆) alkyl, halo, CF₃, OH or OR⁷;
   R⁶ is 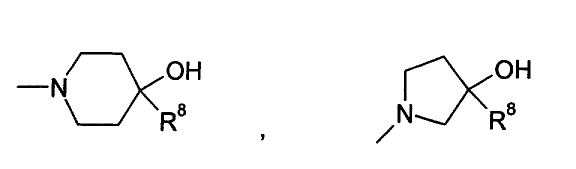 or 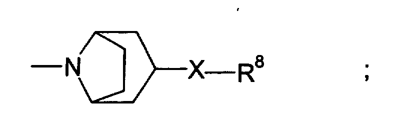
   R⁷ is methyl, ethyl, isopropyl or n-propyl;
   R⁸ is phenyl optionally substituted with up to three substituents independently selected from the group consisting of (C₁-C₆) alkyl, halo and CF₃;
   X is O, S or (CH₂)ₙ; and
   n is 0, 1, 2, or 3.

Specific compounds of formula I that can be used are:
(1S,2S)-1-(4-hydroxyphenyl)-2-(4-hydroxy-4-phenylpiperidin-1-yl)-1-propanol;
(1S,2S)-1-(4-hydroxy-3-methoxyphenyl)-2-(4-hydroxy-4-phenylpiperidino)-1-propanol;
(3R,4S)-3-(4-(4-fluorophenyl)-4-hydroxypiperidin-1-yl)-chroman-4,7-diol;
(1R*,2R*)-1-(4-hydroxy-3-methylphenyl)-2-(4-(4-fluorophenyl)-4-hydroxypiperidin-1-yl)-propan-1-ol;
enantiomers thereof; and
pharmaceutically-acceptable salts of the above compounds and their enantiomers.

The compounds of formula I can be prepared as follows. The compounds of formula I wherein R² and R⁵ are taken together forming a chroman-4-ol ring, and R¹, R³, and R⁴ are hydrogen, can be prepared by one or more of the synthetic methods described and referred to in U.S. Patent No. 5,356,905 to Butler, issued October 18, 1994, which is incorporated herein by reference. The compounds of formula I wherein R² and R⁵ are taken separately, and R¹, R², R³ and R⁴ are hydrogen, can be prepared by one or more of the synthetic methods described and referred to in U.S. Patent No. 5,185,343 to Chenard, issued February 9, 1993; U.S. Patent No. 5,272,160 to Chenard, issued December 21, 1993; and U.S. Patent No. 5,338,754 to Chenard, issued August 16, 1994; all of which are incorporated herein by reference in their entireties. The compounds of formula I can also be prepared by one or more of the synthetic methods described and referred to in U.S. Patent No. 6,046,213 to Chenard et al., issued April 4, 2000; U.S. Patent No. 5,744,483 to Butler et al. issued April 28, 1998; U.S. Patent No. 6,008,233 to Andino et al., issued December 28, 1999; International Patent Publication WO 96/37226 by Pfizer Inc., published November 28, 1996; and International Patent Publication WO 96/06081 by Pfizer Inc., published February 29, 1996. All of the U.S. patents, published applications and cited scientific publications cited herein are incorporated by reference herein in their entireties.

A preferred compound, (1S,2S)-1-(4-hydroxyphenyl)-2-(4-hydroxy-4-phenylpiperidin-1-yl)-1-propanol ((1S,2S) free base), which is designated as CP-101,606, and its tartrate salt, can be prepared as described in U.S. Patent No. 5,272,160, referred to above. The resolution of racemic 1-(4-hydroxyphenyl)-2-(4-hydroxy-4-phenylpiperidin-1-yl)-1-propanol to form the (1S,2S) free base and the corresponding (1R,2R) enantiomer can be carried out as described in U.S. Patent No. 6,008,233, referred to above.

The anhydrous mesylate of the (1S,2S) free base can be prepared as described in U.S. Patent No. 5,272,160, referred to above. The anhydrous mesylate of the (1S,2S) free base, when equilibrated in an 81% relative humidity environment, will convert to the mesylate salt trihydrate of the (1S,2S) enantiomer.

The mesylate salt trihydrate of (1S,2S)-1-(4-hydroxyphenyl)-2-(4-hydroxy-4-phenylpiperidin-1-yl)-1-propanol can be prepared from the (1S,2S) free base as described in the U.S. Patent No. 6,008,233.

Another preferred compound, (3R,4S)-3-(4-(4-fluorophenyl)-4-hydroxy-piperidin-1-yl]-chroman-4,7-diol ((3R,4S) chromanol), can be prepared as described in U.S. Patent No. 5,356,905, and U.S. Patent No. 5,744,483, which are referred to above. The starting materials and reagents required for the synthesis of the (3R,4S) chromanol are readily available, either commercially or according to synthetic methods disclosed in the literature.

The structure of ifenprodil is shown below as Formula II, and may be prepared by methods analogous to those disclosed in U.S. Patent No. 3,509,164. The structure of eliprodil is shown below as Formula III, and may be prepared by methods analogous to those disclosed in U.S. Patent No. 4,690,931.

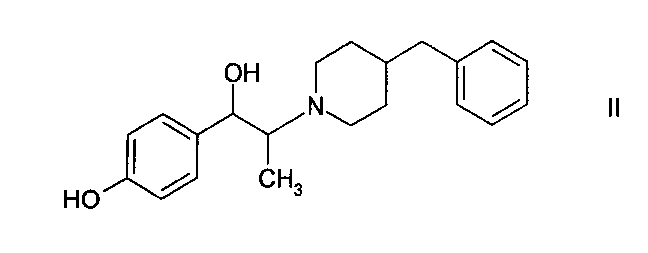

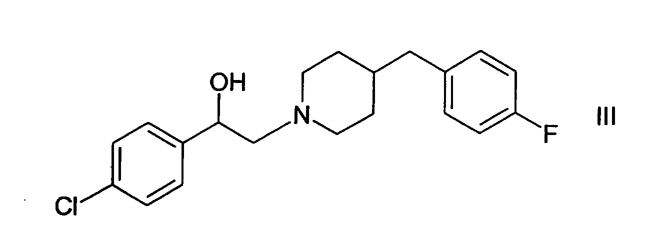

NR2B subunit selective NMDA receptor antagonists useful in the practice of the invention can be used in the form of a pharmaceutically acceptable salt. The expression "pharmaceutically-acceptable salt" is intended to include but not be limited to such salts as the hydrochloride, hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts. The acid addition salts of the compounds used according to the methods of the present invention are readily prepared by reacting the base forms with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

Any other compound that is an NR2B subunit selective NMDA receptor antagonist, including a pharmaceutically acceptable salt thereof, can be used in the methods of this invention. NMDA receptor antagonists having NR2B subunit selectivity that may be used according to the present invention include, e.g., those described in U.S. Patent No. 6,046,213; U.S. Patent No. 5,185,343; U.S. Patent No. 5,272,160; U.S. Patent No. 5,338,754; U.S. Patent No. 5,356,905; U.S. Patent No. 6,046,213; U.S. Patent No. 5,744,483; U.S. Patent No. 6,008,233; WO 96/37226; and PCT publication WO 96/06081. Other NR2B subunit selective NMDA receptor antagonists that may be used according to the present invention are described in WO 97/32581; WO 98/18793; WO 97/23202; EP 0 824 098 A1; EP 0 846 683 A1; and DE 19739331, published November 26, 1998.

Other compounds that are indicated to bind selectively to NR2B NMDA receptor subunits that may be used according to the methods of the present invention are *ifenprodil*, supra, eliprodil (described in U.S. Patent No. 4,690,931), and compounds described in WO 97/23458; WO 97/23216; WO 97/23215; and WO 97/23214.

Compounds that selectively antagonize NMDA receptors comprising an NR2B subunit by specifically binding to the NR2B subunit can be determined by screening compounds for inhibition of NMDA-induced current in recombinant *Xenopus* oocytes co-transfected with the NR1A subunit and the NR2B subunit (see, e.g., Monyer, et al., *Science,* 1992, 256:1217-1221). A compound's activity in inhibiting current in the recombinant cells comprising the NR2B subunit can be compared to its activity inhibiting NMDA-induced current in recombinant *Xenopus* oocytes expressing the NR1 subunit and NR2A, NR2C, and NR2D subunits. (See, Chenard and Menniti, *supra*).

One general method that can also generally predict whether or not a compound has NR2B subunit selectivity, for purposes of the present invention, is a standard competitive binding assay using [³H] radiolabeled racemic CP-101,606 (which contains [³H] (+)-(1S, 2S)-1-(4-hydroxy-phenyl)-2-(4-hydroxy-4-phenylpiperidino)-1-propanol; see, for example, U.S. Patent 6,046,213). If a compound has an IC₅₀ of less than about 5µM for inhibition of racemic [³H] CP-101,606 binding to the NR2B subunit, than the compound has NR2B subunit selectivity for purposes of the present invention. An example of such an assay is as follows.

***Example*** *of NR2B subunit binding assay.* Selectivity of compounds for the NR2B-subunit-containing NMDA receptor can be defined as an affinity for the racemic [³H] CP-101,606 binding site in the forebrain of rats, as described in Chenard and Menniti, *supra.* This affinity is assessed in a radioligand binding assay as described below. Selective compounds are preferably those which displace specific binding of racemic [³H]CP-101,606 from rat forebrain membranes with an IC₅₀ of about ≤ 5 µM.

The binding of racemic [³H] (+)-(1S, 2S)-1-(4-hydroxy-phenyl)-2-(4-hydroxy-4-phenylpiperidino)-1-propanol to rat forebrain membranes is measured as described by Menniti *et al.* (CP-101,606, a potent neuroprotectant selective for forebrain neurons, *European Journal of Pharmacology,* 1997, 331:117-126). Forebrains of adult male CD rats are homogenized in 0.32M sucrose at 4°C. The crude nuclear pellet is removed by centrifugation at 1,000 x g for 10 min, and the supernatant centrifuged at 17,000 x g for 25 min. The resulting pellet is resuspended in 5mM Tris acetate pH 7.4 at 4°C for 10 min to lyse cellular particles and again centrifuged at 17,000 x *g*. The resulting pellet is washed twice in Tris acetate, resuspended at 10mg protein/ml and stored at -20°C until use.

For binding assays, membranes are thawed, homogenized, and diluted to 0.5mg protein/ml with 50mM Tris HCI, pH 7.4. Compounds under study are added at various concentrations followed by racemic [³H] CP-101,606 (specific activity 42.8 Ci/mmol, 5nM final concentration). Following incubation for 20 min at 30°C in a shaking water bath, samples are filtered onto Whatman GFB glass fiber filters using a MB-48R Cell Harvester (Brandel Research and Development Laboratories, Gaithersburg MD). Filters are washed for 10 s with ice cold Tris HCI buffer and the radioactivity trapped on the filter quantified by liquid scintillation spectroscopy. Nonspecific binding is determined in parallel incubations containing 100µM racemic CP-101,606. Specific binding is defined as total binding minus nonspecific binding.

That amount of a neuroprotective agent to be administered to a patient and constituting an "effective amount" will generally depend on the specific circumstances of the patient, including among other factors the patient's sex, weight, age, and general health, as well as the type and severity of the event or condition for which the patient is being treated. The effective amount of a neuroprotective agent will typically be determined by the attending physician using such information in combination with the results of clinical studies and published reports regarding the particular agent. For example, an effective amount of an NR2B selective NMDA antagonist will range from about 0.02 mg/kg/day to about 10 mg/kg/day. Of course, depending on the specific circumstances of the particular patient being treated, as well as the specific neuroprotective agent being administered, dosages outside this range may be required, and these may be determined by the attending physician in view of the particular circumstances.

The neuroprotective agent will generally be administered in the form of a pharmaceutical composition further comprising a pharmaceutically acceptable carrier or diluent as known in the art. Such compositions are generally formulated in a conventional manner utilizing solid or liquid vehicles or diluents as appropriate to the mode of administration. For purposes of oral administration, tablets containing excipients such as sodium citrate, calcium carbonate, and di-calcium phosphate may be employed, along with various disintegrants such as starch, preferably potato or tapioca starch, alginic acid and certain complex silicates, together with binding agents such as polyvinylpyrrolidone, sucrose, gelatin and acacia. Additionally, lubricating agents such as, but not limited to, magnesium stearate, sodium lauryl sulfate and talc are often very useful for tableting purposes. Solid compositions of a similar type may also be employed as fillers in soft elastic and hard filled gelatin capsules. Preferred materials in this connection also include by way of example lactose or milk sugar as well as high molecular weight polyethylene glycols. When aqueous suspensions and/or elixirs are desired for oral administration, the essential active ingredient may be combined with various sweetening or flavoring agents, coloring matter or dyes and, if so desired, emulsifying and/or suspending agents, together with diluents such as water, ethanol, propylene glycol, glycerin and various combinations thereof.

For purposes of parenteral administration, solutions of a neuroprotective compound used in the methods of the present invention are formulated according to standard techniques. For example, solutions of a neuroprotective compound in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for infra-articular, intramuscular and subcutaneous injection purposes. The preparation of all these solutions under sterile conditions is readily accomplished by standard pharmaceutical techniques well known to those skilled in the art.

The present invention further provides a method for identifying whether a patient will benefit from treatment with a neuroprotective agent comprising determining whether the amount of at least one biomarker in a biological sample taken from the patient prior to an initial treatment with the neuroprotective agent is above a certain predetermined minimum threshold value, such that if the amount of the biomarker in the biological sample taken from the patient is above the minimum threshold value, then the patient is primarily identified as a patient who has suffered substantial neurological damage. For example, for S-100b, such a minimum threshold value can be about 0.2 µg/L in serum, particularly where the level of S-100b remains at or above that level for at least 24 hrs. As explained above, however, this value may vary depending on the circumstances of the individual patient.

This method may also comprise determining whether the amount of the at least one biomarker in the biological sample taken from the patient prior to initial treatment with the neuroprotective agent is above a certain predetermined maximum threshold value, such that if the amount of the biomarker in the biological sample taken from the patient is above the maximum threshold value, then the patient suffering from neurological damage is secondarily identified as a patient suffering from such severe neurological damage that the patients expected outcome is considered poor. For example, for S-100b, such a level can be about 1.5 to 2.0 µg/L in serum, and preferably remains at or above that level for at least 24 hrs.

This method will serve as a method to screen patients presenting with possible neurological damage to help distinguish those patients who can substantially benefit from treatment with a neuroprotective agent from those who may only benefit minimally if at all.

The following examples are illustrative only, and not intended to limit the scope of the present invention.

### EXAMPLE 1

### Monitoring Response of Patients Being Treated for Neurological Damage

S-100b levels were measured in the serum of patients suffering from severe contusive-type head trauma. A first group of 198 patients received treatment with CP-101,606 by constant infusion for 72 hours. A second group of 202 patients instead received a constant placebo infusion. Serum samples were collected from the patients at various time points out to 120 hours post-infusion (Figure 1). S-100b levels were determined using a commercially available luminometric immunoassay kit from Byk-Sangtec Diagnostica GmbH & Co. (Dietzenbach, Germany).

S-100b levels in the two groups of patients are presented in Figure 1. S-100b levels were significantly reduced in patients administered CP-101,606 compared to the placebo group.

### EXAMPLE 2

### Monitoring Stroke Patients

Plasma samples were collected from patients suffering from ischemic stroke at baseline, 1, 2, 3, 4, 4.5, 7, 30 and 90 days after injury. S100B was measured in all plasma samples and total concentration (in µg/L) was correlated to clinical outcome measures including 30 and 90 day NIHSS (National Institute of Health Stroke Scale) and infarct volume as measured by diffusion weighted MRI. A logistic regression analysis was performed to examine the relationship between 72 hour S100B levels and NIHSS at 90 days. The model corrected for NIHSS baseline scores. Data showed that patients with 72 hour S100B levels were more likely to have poor NIHSS scores. A correlation analysis between actual infarct volume (as assessed by normalized diffusion weighted MRI) and 72 hour plasma S100B levels was also performed. Diffusion weighted MRI images were acquired at baseline and at 48 hours after stroke lesions. Logistically transformed normalized values (Baseline/48hrs) were compared to logistically transformed 72 hour S100B values. Overall, 72 hour plasma S100B values correlated with infarct volume as measured by DW-MRI.

All patents, patent applications, and publications cited above are incorporated herein by reference in their entirety.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims.

## Claims

1. A method to monitor the response of a patient being treated for neurological damage by administering a neuroprotective agent, comprising the steps of:
(a) determining the amount of at least one biomarker in a first biological sample taken from the patient prior to an initial treatment with the neuroprotective agent;
(b) determining the amount of the biomarker in at least a second biological sample taken from the patient subsequent to the initial treatment with the neuroprotective agent; and
(c) comparing the amount of the biomarker in the second biological sample with the amount of the biomarker in the first biological sample;
such that a detectable reduction in the amount of the biomarker in the second biological sample compared to the amount of biomarker in the first biological sample indicates that the patient is responding positively to the treatment with the neuroprotective agent.

2. The method of claim 1, wherein the neurological damage is a condition or disease, or is caused by a condition, disease or event, selected from the group consisting of cerebral ischemia, cerebral infarction, head trauma, contusion, spinal cord injury, subarachnoid hemorrhage, cerebral hemorrhage, aneurysmal hemorrhage, cardiac infarction, hypoxia, anoxia, surgery, Alzheimer's disease, Parkinson's disease, multiple sclerosis, HIV-related neurodegeneration, cerebellar degeneration, seizure and ataxia.

3. The method of claim 2, wherein the neurological damage is cerebral ischemia, cerebral infarction, head trauma, or spinal cord injury.

4. The method of claim 1, wherein the biomarker is selected from the group consisting of S-100b, neuron-specific enolase (NSE), glial fibrillary acidic protein (GFAP), *tau* protein, haptoglobin, brain creatine kinase, isoprostane, myelin basic protein (MBP), or thrombomodulin.

5. The method of claim 4, wherein the biomarker is S-100b or NSE.

6. The method of claim 1, wherein the neuroprotective agent is selected from the group consisting of an excitatory amino acid receptor antagonist, a metabotropic glutamate receptor antagonists, GABA receptor antagonist; a NAALDase enzyme inhibitor, a calpain inhibitor, a p38 mitogen-activated protein kinase (MAPK) inhibitor, an estrogen enantiomer or derivative, a modulator of nitric oxide production, a calmodulin inhibitor, an adenosine receptor modulator, a purine receptor antagonist, a prosaposin receptor activity stimulator, and a synthetic oxygen carrier.

7. The method of claim 6, wherein the neuroprotective agent is an NMDA receptor antagonist.

8. The method of claim 1, wherein the amount of the biomarker in the biological sample is determined by ELISA, RIA, magnetic resonance spectroscopy, HPLC or mass spectrometry.

9. An improvement to a method for treating a patient suffering from neurological damage by administration of a neuroprotective agent, wherein the improvement comprises monitoring the level of at least one biomarker in a biological sample taken from the patient at one or more time points during treatment with the neuroprotective agent so as to determine whether an effective amount of the neuroprotective agent is being administered to the patient.

10. A method for identifying whether a patient will benefit from treatment with a neuroprotective agent, comprising determining whether the amount of at least one biomarker in a biological sample taken from the patient prior to an initial treatment with the neuroprotective agent is above a predetermined minimum threshold value, such that if the amount of the biomarker in the biological sample taken from the patient is above the minimum threshold value, then the patient is primarily identified as a patient who has suffered neurological damage.
